# EUROPEAN PATENT APPLICATION

(11) **EP 4 810 621 A1**
(43) Date of publication of application: **23.09.2026**
(21) Application number: 25164751.7
(22) Date of filing: 19.03.2025
(51) Int. Cl.: C12P 19/04, C12P 39/00, C12N 1/12, C12N 1/20

(54) **METHOD FOR COCULTIVATION OF MICROORGANISMS AND ENGINEERED LIVING MATERIAL**

(71) Applicant: Max-Planck-Gesellschaft zur Förderung der Wissenschaften e.V., 80539 München (DE)
(72) Inventor: VIGNOLINI, Silvia, 14469 Potsdam (DE); YU, Kui, Suzhou 215557 (CN); CHUA, Sing Teng, 3000 Helsingør (DK)
(74) Representative: Manitz Finsterwald Patent- und Rechtsanwaltspartnerschaft mbB

(57) **Abstract**

The present invention concerns a method for cocultivation of microorganisms in particular for producing bacterial cellulose and biomass including the steps of: (a) Providing a liquid culture of at least a first microorganism which is an oxygen producing microorganism; (b) Providing a liquid culture of at least a second microorganism which is capable of producing bacterial cellulose; (c) Providing a dispersant; (d) Preparing a mixture of the liquid cultures of step (a), step (b) and of the dispersant provided in step (c); (e) coculturing the mixture obtained in step (d) to generate an engineered living material, as well as a bioreactor comprising the engineered living material. In another aspect, the present invention concerns an engineered living material comprising a first microorganism which is an oxygen producing microorganism, and a second microorganism which is capable of producing bacterial cellulose, and a dispersant. Further, the present invention concerns an engineered living material comprising a first microorganism which is an oxygen producing microorganism, a second microorganism which is capable of producing bacterial cellulose, and a dispersant.

## Description

The present invention concerns a method for the cocultivation of microorganisms and an engineered living material.

### Background

Bacterial cellulose is a biopolymer with remarkable properties. The biopolymer owes its exceptional mechanical robustness to the high crystallinity of the cellulose polymer that makes up the individual fibres and their layered arrangement. Beyond its mechanical strength, bacterial cellulose has attracted attention for its functional bio-composite properties, including high purity, high porosity, excellent water retention, self-regeneration capabilities, biocompatibility and biodegradability, which position it as a versatile, beneficial and environmentally friendly material in various applications. These include acoustic or filter membranes, biosensors, biomedical devices, air purifiers, artificial tissues for vascularisation or treatment of skin lesions, waste water treatment, substitutes for conventional plastics and even hydrogen production. Conventional production methods cannot deliver bacterial cellulose in commercial quantities due to inefficient processes and high production costs. Process improvements for large-scale production are therefore required.

Bacterial cellulose (BC) can be obtained by bacterial fermentation under mild temperatures. At present, bacterial cellulose is usually grown as floating pellicles at an air-liquid interphase of a growing medium.^{[1]} In general, liquid cultures comprise a dispersion of microorganisms in a liquid medium, including dissolved or liquid additives and nutrients, ensuring optimum growing conditions. Diffusion limitation of nutrients and low oxygen solubility in liquid media either limit growth or make aeration by mixing inevitable. Conventionally, bacterial cellulose is grown under static as well as agitated culture conditions. However, both static as well as agitated culture conditions are subject to major disadvantages, inhibiting the implementation of large-scale processes.

Conventional bacterial cellulose (BC) has multiple advantages. However, for certain applications including but not limited to food, construction, waste water treatment and biomedicine, further functionalisation and homogeneous incorporation of components possessing functional characteristics is required. In particular, insertion of components providing high electrical conductivity, biocompatibility, photosynthetic or magnetic properties can be necessary to provide highly efficient BC composites designed for target applications.

However, the diffusion of such functional components inside BC is limited to low molecular weight molecules or to nanoscale particles for conventional culture methods. When it comes to macromolecules and microparticles, such as organic polymers, inorganic particles and microorganisms, the diffusion becomes very slow and therefore such components cannot be integrated into the BC network homogeneously. These composites are, hitherto, limited in terms of physical thickness of the bacterial cellulose. To enhance the impregnation depth and speed, energy-consuming vacuum filtration procedure or other treatments with external forces as well as ex situ impregnation are needed to form functionalised BC composites.

Conventional static culture methods for obtaining bacterial cellulose require a long culture period and elevated work input and are thus too expensive to be of interest from an economical point of view. Furthermore, in a static incubation process, the growth of bacterial cellulose is restricted to the air-liquid interface of the growing medium due to diffusion limitations of additives and oxygen. Therefore, the production of bacterial cellulose in a static culture is restricted to thin films, in particular, with regard to the above-mentioned functional adaptations..^{[2]}

Culture techniques involving agitation have been proposed to overcome those drawbacks. While an agitated culture process eliminates mass transport limitations of nutrients and allows for oxygen to diffuse better into the medium, thereby accelerating BC production, it induces the formation of BC spheroids, deteriorating the properties and quality of the resulting BC material. A key disadvantage of agitation is the formation of loose bacterial cellulose, which does not self-assemble into a dense or complete pellicle with mechanical integrity. ^{[3]}

It is therefore an objective of the present invention, to provide a fully scalable method for the industrial cocultivation of a first and a second microorganism in order to obtain an engineered living material containing a symbiotic collective of microorganisms, which produces bulk bacterial cellulose. The engineered living material can be manufactured with a tailored material geometry and allows for a homogeneous incorporation of microparticles into bacterial cellulose via in-situ fermentation under mild and facile conditions.

### Summary of the invention

Specifically, the objective of the present invention is achieved by a method for cocultivation of microorganisms according to claim 1 and an engineered living material according to claim 21. Preferred embodiments of the present invention are the subject-matter of dependent claims and described in the following.

The method according to the invention comprises the steps (a) of providing a liquid culture of at least a first microorganism, which is an oxygen-producing microorganism, (b) providing a liquid culture of at least a second microorganism, which is capable of producing bacterial cellulose, (c) providing a dispersant, (d) preparing a mixture of the liquid cultures of step (a), step (b) and of the dispersant provided in step (c) and coculturing the mixture obtained in step (d) to generate an engineered living material.

The engineered living material according to the invention comprises a first microorganism, which is an oxygen producing microorganism, a second microorganism which is capable of producing bacterial cellulose, and a dispersant. The engineered living material is preferably obtainable by the method according to the present invention.

Accordingly, the present invention applies a combination of synthetic biology and materials science, which has opened up a revolutionary way to produce conventional materials and chemicals, but has also led to an entirely new class of materials called living materials. Living materials are defined as materials composed of living cells, which represent, assemble or represent and assemble at the same time the material itself. Building on this concept, engineered living materials can be designed by targeted selection of microorganisms and the creation of artificially designed symbiosis under controlled cultivation conditions. This approach allows for the modulation of the functional performance of the engineered living material.

In general, an engineered living material is a composite material that integrates living cells into a structural matrix to provide functional properties. In the engineered living material of the present invention, a symbiosis between the first and second microorganisms is artificially generated. The particular symbiotic relationship is characterized in the first microorganism providing oxygen to the second microorganism, which produces a network of bacterial cellulose nanofibres, in which the first microorganism and the second microorganism are distributed. Homogenous distribution of oxygen generating sites within such coculture is supported by the dispersant and overcomes limitation of oxygen diffusion, even when the coculture is kept statically motionless. This allows the cellulose fibres in the culture to grow undisturbed, resulting in the formation of stable fibres with a high degree of polymer crystallinity. The BC fibre network incorporates the first microorganism, which forms cavities within the resulting fibre network.

In the method of the present invention, the dispersant prevents a sedimentation of the first microorganism during an early stage of cocultivation, i.e. before bacterial cellulose is formed in an amount which is sufficient for preventing such a sedimentation. Accordingly, the effect of the dispersant to stabilize the distribution of the first microorganism throughout the bulk of the mixture provided in step (d). Therefore, oxygen producing sites are available throughout said bulk. In consequence, the second microorganism can produce the bacterial cellulose not only at an air-liquid interphase but also throughout the bulk of the mixture. In other words, the dispersant supports the symbiotic interaction of the first and second microorganisms during an early cultivation stage. Remarkably, no agitation of the medium is required for this symbiotic interaction and therefore the process of the present invention is easy to scale up and requires less energy input than agitation-based approaches.

When the method is carried out in an industrial bioreactor, the engineered living material may be used as a feedstock for the production and extraction of valuable biomass, including bacterial cellulose and nanocellulose as well as optionally further compounds of interest, such as antioxidants, vitamins, particularly vitamin B12, pigments, particularly β-carotene and copper-chlorophyllin pigments, Eicosapentaenoic acid (EPA), docosahexaenoic acid (DHA), proteins, biochar and/or agricultural bio-stimulants. The first and/or the second microorganisms may be engineered to produce specific compounds of interest, such as the ones mentioned above.

The first microorganism provided within step (a) is preferably a photosynthetic microorganism. An oxygenic photosynthetic microorganism is capable of transforming inorganic molecules into energy-rich assimilates and oxygen, using the energy of light of suitable wavelength and intensity. In addition to oxygen, such a photosynthetic first microorganism can therefore also provide nutrients for the second microorganism, thereby further supporting their symbiotic interaction. Aerobic respiration of a second microorganism producing carbon dioxide as well as fermentation processes towards gluconic, acetic or lactic acid can undesirably lower the pH value of a culture and thereby affect its viability and longevity. The photosynthetic first microorganism uses carbon dioxide for photosynthesis. The photosynthetic first microorganism can thus remove carbon dioxde leading to an increase of the pH value of a coculture, which counter balances reduction of pH and improves viability of a symbiotic coculture.

Even more preferably, the photosynthetic microorganism is at least one of a microalgae, preferably eukaryotic microalgae, such as *Phaeodactylum tricornutem,* or *Chlamydomonas reinhardtii,* or one of the Chlorella genus of the division Chlorophyta, such as *Chlorella vulgaris,* or *Chlorella sorokiniana,* or a bacteria, such as cyanobacteria, preferably from Phylum Cyanobacteria, such as Chroococales, Nostocales, or Spirulinales, or a combination thereof. The oxygen provided by a photosynthetic microorganism supports growth of an oxygen requiring second microorganisms in a symbiotic coculture. As the first microorganism provides the necessary oxygen for its symbiotic partner while removing waste products of the symbiotic partner, such as carbon dioxide by photosynthesis, agitating of the coculture is no longer necessary. This simplifies the process and reduces the costs thereof. Further, without agitation the bacterial cellulose produced can form an optimal network with long polymer chains and excellent mechanical properties.

In one embodiment of the present invention, the photosynthetic microorganism provided in step (a) may be a microalga. Microalgae typically have a high biodiversity allowing for flexibly adapting choice of species to the needs of a second microorganism in a symbiosis, whereby viability of a coculture can be improved. Preferably, the photosynthetic microorganism is a eukaryotic microalga, which even more preferably is at least one of *Phaeodactylum tricornutem* or *Chlamydomonas reinhardtii.* Alternatively, the photosynthetic microorganism may be a microalga of the Chlorella genus of the division Chlorophyta, such as *Chlorella vulgaris* and/or *Chlorella sorokiniana.* The beforementioned microalgae represent a potential feedstock for a variety of valuable metabolites and downstream exploitation of such compounds. Valuable compounds of interest provided by microalgae include for example antioxidants, vitamins, particularly vitamin B12, pigments, particularly β-carotene and copper-chlorophyllin pigments, Eicosapentaenoic acid (EPA), docosahexaenoic acid (DHA), proteins, biochar and/or agricultural bio-stimulants.

In another embodiment, the first microorganism provided in step (a) may be a bacteria, such as cyanobacteria. The first microorganism can be at least one of Chroococales, Nostocales, Spirulinales or a combination thereof. Cyanobacteria provide a specific combination of photopigments including for example chlorophyll a, β-carotene, phycocyanin, zeaxanthin and/or echinenone, allowing photosynthesis by absorption in specific spectrum frequencies of light. Culture conditions as well as choice of organism can be flexibly adjusted and optimized to improve viability of a coculture of a first microorganism and a second microorganism. Furthermore, the production of compounds of interest may thereby be customized to metabolites specific for such bacteria.

The second microorganism provided in step (b) is capable of producing bacterial cellulose. Preferably, the second microorganism is at least one bacteria from the group consisting of *Acetobacter xylinum, Komagataeibacter hansenii,* and *Acetobacter pasteurianus* as well as combinations thereof. These bacteria are capable of producing bacterial cellulose with a high crystallinity. Individual fibres of the polymer are arranged in a layered structure, providing high robustness and mechanical strength of the resulting material. In particular the second microorganism may be *Komagataeibacter hansenii..* Preferably, when the second microorganism is *Komagataeibacter hansenii,* the first microorganism is a microalga selected from at least one of the group consisting of *Phaeodactylum tricornutem, Chlamydomonas reinhardtii. Chlorella vulgaris* and/or *Chlorella sorokiniana.* Exemplary experiments, which are described in detail below, have shown successful cocultivation of the beforementioned microalgae along with *Komagataeibacter hansenii* under engineered cultivation conditions, albeit the present invention is not limited to these specific combinations. After short incubation times, all microalgal cells in planktonic state are covered by bacterial cellulose fibres and dispersed homogeneously within the bacterial cellulose network.

As mentioned already above, by utilizing a dispersant, stabilization of a mixture during an initial stage is achieved, when a mixture of liquid cultures of at least a first microorganism and at least a second microorganism are prepared in step (d). Preferred dispersants provide good biocompatibility with the at least first and second microorganisms and prevent their aggregation and sedimentation. Specifically, a dispersant may prevent sedimentation of the first microorganism that provides oxygen generating sites in order to achieve optimum distribution of oxygen within the coculture. The dispersant provided within step (c) may preferably be in the form of colloidal particles, having a particle size of 1.0 µm or less, when determined by transmission electron microscopy (TEM) in accordance withlSO 21363:2020. Particularly preferable, the dispersant may be in the form of charged particles comprising ionic groups on their surface. Such charged particles can form a stable fluid with a low yield stress and furthermore support interaction of the dispersant with a surface of solids and/or cell walls and improve particle (here microorganism) separation by electrostatic repulsion. The yield stress is the minimum shear stress required to initiate flow in a material. As used herein, a fluid with a low yield stress refers to a fluid having a yield stress of greater than 0 Pa, in particular equal to or greater than 0.05 Pa. The yield stress can be measured in accordance with DIN 53019-1:2008-09. Further, in this context, a stable fluid refers to a fluid which maintains its properties over an extended period of time, in particular without sedimentation of e.g. charged particles or microorganisms. Alternatively, the yield stress of a fluid can be determined by dynamic light scattering (DLS). Dynamic light scattering allows for determination of particle sizes in a fluid according to ISO 22412:2017 and of yield stress of ultra-low yield stress fluids as described in reference [4]. The dispersant may comprise inorganic particles of biocompatible metal oxides. Such metal oxides include for example titania, alumina, silica, magnesia, lime and/or iron oxide. The dispersant may optionally comprise organic particles in addition to inorganic particles or exclusively organic particles. Organic particles representing suitable dispersants include polymeric compounds. Suited polymeric compounds include biopolymers as for example polysaccharides and/or polypeptides. In particular, cellulose nanocrystals provide good biocompatibility and dispersing properties over a long time period. Suited cellulose nanocrystals are for example obtainable by acidic hydrolysis of freeze dried bacterial cellulose fibres or acidic hydrolysis of freeze dried and purified plant derived cellulose fibres. In particular bacterial cellulose nanocrystals are preferred, as in comparison to plant derived cellulose, they are more chemically pure, constituted of longer, thinner microfibrils and have a higher water retention capacity. The concentration of the dispersant may be adjusted to the viability of the first and/or the second microorganism, in order to ensure stable cultivation conditions, and to the time required for the initial cultivation stage during which sedimentation needs to be prevented. From the viewpoint of preventing sedimentation of the first microorganism during an early stage of cocultivation, it is preferable to have the content of the dispersant in the mixture prepared in step (d) in an amount of 0.005 w/v% or more, more preferably of 0.010 w/v% or more and even more preferably of 0.030 w/v% or more. Considering that a dispersant can have an influence on the pH value of the culture medium, it is preferable to not use an excessive amount of dispersant, as this may have a negative influence on the viability of the microorganisms. In the context of cell viability, it is therefore preferable to adjust the amount of the dispersant so that cell viability and in particular the production of bacterial cellulose by the second microorganism is maintained. More preferably, in this context the content of the dispersant in the mixture prepared in step (d) is 0.10 *w*/*v%* or less, even more preferably 0.06 *w*/*v%* or less, and even further more preferably 0.05 *w*/*v%* or less. The content of the dispersant, in particular of cellulose nanocrystals, in the mixture obtained in step (d) is preferably 0.005 w/v% or more and 0.10 *w*/*v%* or less, more preferably 0.010 w/v% or more and 0.06 *w*/*v%* or less, and even more preferably 0.030 w/v% or more and 0.05 *w*/*v%* or less. A dispersant content, in particular when it is cellulose nanocrystals, within these ranges allows a good balance of cell viability of the first and second microorganisms and of a stabilizing effect of preventing the microorganisms from sedimentation at an early cultivation stage. Decrease in viability or bacterial cellulose production may result from a drop of pH value of the culture medium, which may be caused by release of protons of the hydroxide groups present at the surface of a dispersant, such as cellulose nanocrystals in aqueous media.

The first and the second microorganisms may be inoculated, precultured and provided in liquid culture during steps (a) and (b), respectively. A liquid culture of microorganisms is a dispersion of microorganisms in a liquid culture medium, including nutrients and optionally additives. The culture medium formulation as well as the composition of optional additional additives may be specifically adapted to ensure optimum viability of selected microorganisms. When the first microorganism provided in step (a) is Chlamydomonas, the liquid culture medium preferably is a tris-acetate-phosphate (TAP) containing medium. For bacterial cellulose producing bacteria, i.e. the second microorganism, the liquid culture medium is preferably a Hestrin-Schramm (HS) medium. Further preferred culture media for specific microorganisms are generally known in the art. Choice of medium and its nutrient composition is based on the choice of the first and/or second microorganism and the invention is not limited to the examples for culture media mentioned herein, even though the specifically mentioned media TAP and HS are particularly preferred.

Particularly, the first and the second microorganism may initially be provided in different liquid culture media. In that case, a mixture of different culture media is obtained during step (d) when preparing a mixture thereof. The ratio of a first liquid culture medium for a first microorganism to a second liquid culture medium for a second microorganism may be adjusted to the viability of the first and/or the second microorganism. When the first microorganism is provided in a first medium, such as TAP medium, and the second microorganism is provided in a second medium, such as a HS medium, the medium volume ratio of first to second medium is preferably greater than or equal to 30% and less than or equal to 50% and more preferably greater than or equal to 35% and less than or equal to 45%. Depending on the first and second microorganisms and the exact composition of the first and second medium, the ratio of these media may vary.

When mixing the first and second microorganisms and the dispersant in step (d), further additives and nutrients may also be added during step (d). The mixture prepared in step (d) may for example further comprise a pH adjusting additive. In particular, one additive selected from the group consisting of buffers, acids, bases and/or mixtures thereof may be added within step (d) of the described method. The pH value of the mixture can be adjusted while preparing the mixture within step (d) and/or during coculturing the mixture in step (e). The additive preferably comprises an alkaline agent. In general, the pH value may be adjusted to ensuring suitable growth conditions and viability of a first and/or a second microorganism of the coculture. While this may vary depending on the specific microorganisms, a pH value in the range of 4.0 to 7.5 is preferred for many instances. More preferably, the pH value is adjusted to the range of 4.5 to 6.5 and even more preferably to the range of 5.0 to 6.0. Suitable alkaline agents may be chosen from at least one of the group consisting of NaOH, KOH, LiOH, NH₄OH, Ca(OH)₂, Ba(OH)₂, Na₂CO₃, K₂CO₃, NaHCO₃ and/or KHCO₃ in order to counter for example a pH value lowering effect of the dispersant. The overall pH value of the co-culture nutrient medium can be adapted to sustaining healthy proliferation of microorganisms as well as consistent cellulose production. A pH value of around 6 can maximize cellulose production by bacteria. Photosynthetic activity of a photosynthetic microorganism can be enhanced from a pH value of 7 to a pH value of 8. In particular, the additive may be NaOH, which has shown good biological compatibility.

Conditions during step (e) of coculturing the mixture may be optimized for large scale production of the engineered living material and of further compounds of interest resulting therefrom. In the method according to the present invention, physico-chemical conditions, such as the nutrients, salinity and pH value of the medium also availability of oxygen and carbon dioxide, temperature and light conditions are preferably adjusted during coculturing.

Culturing of the first microorganism, either as an optional preculture in step (a) or as a coculture in step (e), may be carried out under light of at least 10 µmo·m⁻²·s⁻¹ photons, preferably from 26 to 400 µmol m⁻² s⁻¹ photons. Incubation may be carried out under natural light and/or artificial light using one or more light sources. Frequency maxima of a light source or of a combination of light sources may be adapted to absorption maxima of photoactive pigments present in the first microorganism, in order to enhance photosynthetic activity of said microorganism. Thereby, supply of oxygen to a second microorganism can be increased. Furthermore, specific light/dark or high light/low light periods may contribute to increasing total biomass production, i.e. bacterial cellulose and optional further compounds of interest. Growth of the engineered living material may be accelerated by applying 12h/12h light/dark conditions. 16h/8h light-dark periods may for example specifically improve cell growth when the first microorganism is *Chlorella vulgaris.* When the first microorganism is *Chlamydomonas reinhardtii,* light/dark conditions may be 12h/12h or illumination may even be continuous. A light source might contribute to temperature increase, which may be compensated by using and regulating an additional cooling and/or heating system for temperature control.

Preferably, during step (e) of coculturing, temperature fluctuations are avoided and kept below 10 °C, preferably below 5 °C and even more preferably below 3 °C. This may be realized by using and regulating an additional cooling and/or heating system for temperature control. Preferably, the temperature within step (e), is from 1 to 75 °C, preferably from 10 to 50 °C and more preferably from 25 to 30 °C. In the aforementioned preferred temperature ranges, enzyme activity as well as the production of biomass can be increased, while at the same time undesired denaturation of proteins is avoided.

The method of the present invention may preferably further comprise a step (f) of supplying nutrients to the engineered living material. The supplied nutrients may comprise yeast extract, phosphates, carbohydrates in particular glucose, essential amino acids, water soluble salts of trace elements and/or mineral nutrients. Nutrients may also be supplied, by adding additional, freshly prepared culture medium or a mixture of the preferred culture media for the first and the second microorganism. In case a mixture of different culture media is supplied, the ratio of media may be adapted to the viability of a first microorganism and/or a second microorganism. In case toxic metabolites might accumulate in liquid media during cocultivation, the entire medium or at least parts of it can be replaced. Within a batch process, this can be done in intervals. As part of a continuous process, defined quantities of a liquid medium can be exchanged on an ongoing basis.

The method of the present invention can be carried out within an incubator or bioreactor, in particular to produce an engineered living material according to the invention and preferably also biomass, such as cellulose material, e.g. bacterial cellulose and nanocellulose, and optionally further compounds of interest.

The engineered living material, preferably when it is one that is obtainable by the method according to the invention, can grow to a bulk thickness of up to several centimetres, at least 1 mm, preferably of at least 2 mm, more preferably of at least 1 cm and even more preferably of at least 2.5 cm. In particular, the dimensions of the engineered living material can be determined by the inner dimension of a culture vessel. The maximum bulk thickness is not particularly limited and depends on the geometry of the culture vessel; the maximum bulk thickness may be 50 cm or less, 40 cm or less, 30 cm or less, or 20 cm or less. The first microorganism and the second microorganism are preferably distributed throughout the entire thickness of the material. Further preferably, the first and the second microorganism are distributed homogeneously throughout the entire thickness of the material. The increase of achievable bulk thickness in combination with the mechanical robustness allow production of the engineered living material in an unprecedented shape variety and the utilization of a much large volume for producing biomass, such as bacterial cellulose and further compounds of interest mentioned above. As no external agitation is required, the method of the invention can be scaled for industrial application and carried out in an energy-efficient and cost-effective manner.

According to a particularly preferred embodiment of the present invention, the engineered living material has a substantially homogenous functionalization throughout its bulk, i.e. a functionalisation agent is substantially homogenously distributed throughout the bulk. The functionalisation agent is preferably a macromolecular compound, microparticles or a mixture thereof. Examples are organic polymers and inorganic particles. In general, the functionalisation agent can be added during any of steps (a), (b), (c), (d) and (e) of the method for cocultivation of microorganisms according to the present invention, wherein, if added in step (e), it is added at an early stage of the coculturing, i.e. while the mixture is still liquid, specifically prior to gelation due to production of bacterial cellulose. Thereby, functionaisation is realized in situ, allowing for a substantially homogenous distribution of the functionalisation agent throughout the bulk material without requiring energy-consuming vacuum filtration procedures or other treatments based on applying external forces.

A suitable bioreactor according to the invention comprises the engineered living material for the production of compounds of interest. These compounds of interest are bacterial cellulose and/or nanocellulose that may be produced by the second microorganism but also further compounds of interest which are preferably at least one selected from the group consisting of antioxidants, vitamins, particularly vitamin B12, pigments, particularly β-carotene and copper-chlorophyllin pigments, Eicosapentaenoic acid (EPA), docosahexaenoic acid (DHA), proteins, biochar and/or agricultural bio-stimulants and combinations thereof.

In particular, bacterial cellulose can be obtained by a method for cocultivation of microorganisms according to the invention. During cocultivation, the bacterial cellulose fibre network incorporates the first and the second microorganism. Cells of the first and/or the second microorganism thereby form cavities within the resulting fibre network, lowering the fibre density of the bacterial cellulose network. Bacterial cellulose can be cleaned and/or dried for subsequent processing or use. By doing so, cells of a first and/or a second microorganism can be removed and cavities remain as free gaps. It was observed that such bacterial cellulose has an increased elongation at break while at the same time the tensile strength thereof is reduced, compared to conventional bacterial cellulose. Accordingly, bacterial cellulose obtainable by the method and/or by the engineered living material according to the present invention constitutes a further aspect of the present invention.

When the production of a compound of interest within the engineered living material is carried out in a bioreactor, the method may further comprise a step, wherein the compound of interest is obtained from the engineered living material, in particular by subjecting the engineered living material to a step of macerating, extracting, separating and/or purifying components of the engineered living material.

The bioreactor is preferably a photobioreactor. In general, the bioreactor may be any cultivation system suited for cultivation of photoautotrophic organisms according to the method described herein. Non-limiting examples include glass fermenters, plate shaped photobioreactors, tubular photobioreactors, horizontal photobioreactors, foil photobioreactors or open systems including vessels, tanks, tubs or ponds. In particular, photobioreactors designed or adjusted for statically motionless incubation periods are preferred.

In the following, the present invention is further described within examples and figures. These examples and figures are not limiting to the present invention. Within the drawings, the following is shown:
FIG. 1 schematically shows steps (a), (b), (d) and (e) of an embodiment of the method according to the invention.
FIG. 2 illustrates the stability of a suspension of microalgae in the absence and presence of increasing amounts of a dispersant.
FIG 3A illustrates the status of bacteria culture after 4 days of static incubation.
FIG. 3B illustrates the status of microalgae culture after 4 days of static incubation.
FIG. 3C illustrates the status of bacteria and microalgae coculture after 4 days of static incubation.
FIG. 3D illustrates the status of microalgae and BCNC mixture after 4 days of static incubation.
FIG. 3E illustrates the status of and bacteria and microalgae coculture in the presence of BCNC (not shown) after 4 days of static incubation.
FIG. 4A illustrates variations in wet thickness and cell viability of an engineered living material depending on the content of a dispersant.
FIG. 4B illustrates variations in wet thickness and cell viability of an engineered living material depending on the ratio of culture media.
FIG. 4C illustrates variations in wet thickness and cell viability of an engineered living material depending on the content of an alkaline agent.
FIG. 5A shows the development during the first four days of cocultivation regarding the material morphology of an engineered living material (photographs), cell morphology of the engineered living material (microscope) and morphology of supernatant (microscope).
FIG. 5B shows a scanning electron microscope image (SEM) of a microalgal cell entangled in a fibrous bacterial cellulose network.
FIG. 5C shows a scanning electron microscope image (SEM) of a microalgal cell covered by bacteria and bacterial cellulose fibres.
FIG. 5D shows the development of the wet thickness of an engineered living material during the first four days of cocultivation.
FIG. 5E shows the development of dry weight of an engineered living material during the first four days of cocultivation.
FIG. 5F shows the development of biomass of an engineered living material during the first four days of cocultivation.
FIG. 5G shows the logarithmic development of colony forming units (CFU) of bacteria in an engineered living material during the first four days of cocultivation.
FIG. 5H shows the development of the cell density of microalgae in an engineered living material during the first four days of cocultivation.
FIG. 5I shows the development of cell viability of microalgae in an engineered living material during the first four days of cocultivation.
FIG. 6A illustrates schematically the fibre density of bacterial cellulose in a conventional culture comprising solely a second microorganism (not shown).
FIG. 6B illustrates schematically the fibre density of bacterial cellulose within an engineered living material according to the invention, comprising a first microorganism (shown as circles) and a second microorganism (not shown).
FIG. 6C shows a scanning electron microscope (SEM) image of the fibre density of a dried sample of bacterial cellulose grown in a conventional culture comprising solely a second microorganism.
FIG. 6D shows a scanning electron microscope (SEM) image of the fibre density of a dried sample of an engineered living material according to the invention.
FIG. 7 shows a comparison of tensile properties of a dried sample of bacterial cellulose grown in a conventional culture comprising solely a second microorganism and of a dried sample of an engineered living material according to the invention (BC-based bio-composite).
FIG. 8 illustrates four examples of geometries of the engineered living material demonstrating accessible shape diversity.
FIG. 9A shows the BC-based bio composites (engineered living material) being revived for one, four and seven days by application of fresh medium after starvation.
FIG. 9B shows optical microscope images of the BC-based bio-composites (wet) after 4 days of revival including aggregations formed by microalgae cells.
FIG. 9C shows a sample of the BC-based bio composite (engineered living material) after 1 month of starvation and after 4 days of revival.
FIG. 9D illustrates the regenerative properties of BC-based bio-composites. The material can be regenerated by further incubation after being cut into two pieces or dipped a rectangular hole.
FIG. 9E illustrates scalability of BC-based bio-composites: Sixteen pre-grown building blocks of BC-based bio-composites can be further incubated into a larger piece of material.

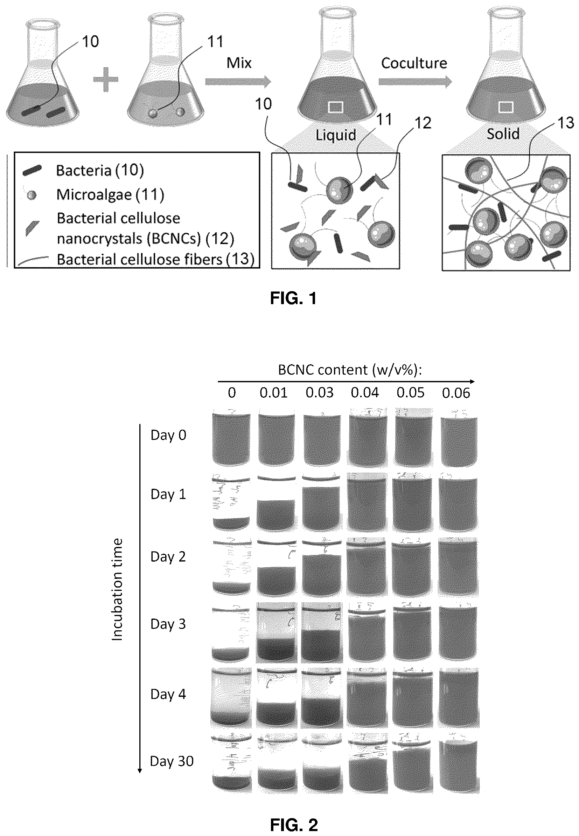

FIG. 1 schematically shows an embodiment of the method according to the invention. A liquid culture of a first microorganism capable of producing oxygen is provided, which in this case is a microalga 11 (step a). An additional liquid culture of a second microorganism, here bacteria 10, capable of producing bacterial cellulose, is provided (step b). Cellulose nanocrystals 12 are provided as a dispersant (step c, not explicitly shown in FIG. 1) and a mixture of the beforementioned is prepared (step d). The bacterial cellulose nanocrystals 12 act as dispersant ensuring homogeneous distribution of microalga 11 and bacteria 10 cells within the liquid phase of the dispersion. During coculturing (step e), an engineered living material forms within the liquid, comprising a network of bacterial cellulose 13 fibres, wherein bacteria 10 and microalga 11 cells are captured.

Bacterial cellulose is usually produced by inoculating cellulose-producing bacteria, such as *Komagataeibacter hansenii* into Hestrin-Schramm (HS) medium. As the production of bacterial cellulose requires oxygen, the bacterial cellulose is formed as a solid flat pellicle at the air-liquid interface under static incubation, due to gas diffusion limitations. Introduction of oxygen-generating sites by adding an oxygen generating first microorganism without a dispersant, allows for obtaining a coculture only when agitating the mixture, while during static incubation cells of the oxygen producing microorganism would sediment before encapsulation in a bacterial cellulose network.

The formation of the engineered living material according to the invention requires homogeneous distribution of a first microorganism generating oxygen and a second microorganism producing a network of cellulose fibres in a suspension, in particular during an initial state. Only if both microorganisms remain suspended until a bacterial cellulose network is formed, homogeneous distribution of oxygen generating sites and cellulose producers within the symbiotic relationship can be ensured.

To avoid sedimentation of an oxygen producing first microorganism, a dispersant is introduced into the coculture system. The stabilized suspension of an oxygen producing first microorganism provides enough time for the bacterial cellulose producing second microorganism to secret sufficient solid cellulose fibres to encapsulate an oxygen producing first microorganism homogeneously throughout the volume of the cellulose network.

In the following, bacterial cellulose derived nanocrystals are used as an example for a dispersant. In general, any dispersant having adequate biocompatibility and allowing for a high viability of a first and/or a second microorganism may be used in terms of the invention. Bacterial cellulose nanoparticles may be obtained by acid hydrolysis of freeze-dried bacterial cellulose fibres, as described in the experimental section below. When bacterial cellulose nanocrystals are added as a dispersant to a liquid culture of an oxygen producing first microorganism, sedimentation may be prevented for more than 30 days, as demonstrated by the results shown in FIG. 2.

Six samples of liquid cultures of microalga *Chlamydomonas reinhardtii* have been prepared, comprising increasing amounts of bacterial cellulose nanocrystals (BCNC) as a dispersant. The samples were cultured under statically motionless conditions for a total duration of 30 days. Obtained results are shown in FIG. 2. In the absence of a dispersant (0 w/v% BCNC), cells completely sedimented at the bottom of the flask already after one day. The sample with a content of 0.01 w/v% of BCNC showed partial sedimentation after just one day, which progressed until day 30 of incubation. The sample with a content of 0.03 w/v% of BCNC showed some sedimentation after day one and day two, which also progressed until day 30 of incubation, but with a much slower rate compared to samples having 0 w/v% BCNC and 0.03 w/v% BCNC. Samples with a content of 0.04 w/v% and 0.05 w/v% of BCNC, however, showed almost no sedimentation until day 4 of incubation. With a content of 0.06 w/v% of BCNC, an optically homogeneous suspension was found even after an incubation period of 30 days. As discussed above, the duration and quality of the stabilization of a suspension by the content of a dispersant must be weighed against the compatibility with the viability and productivity of the microorganisms involved.

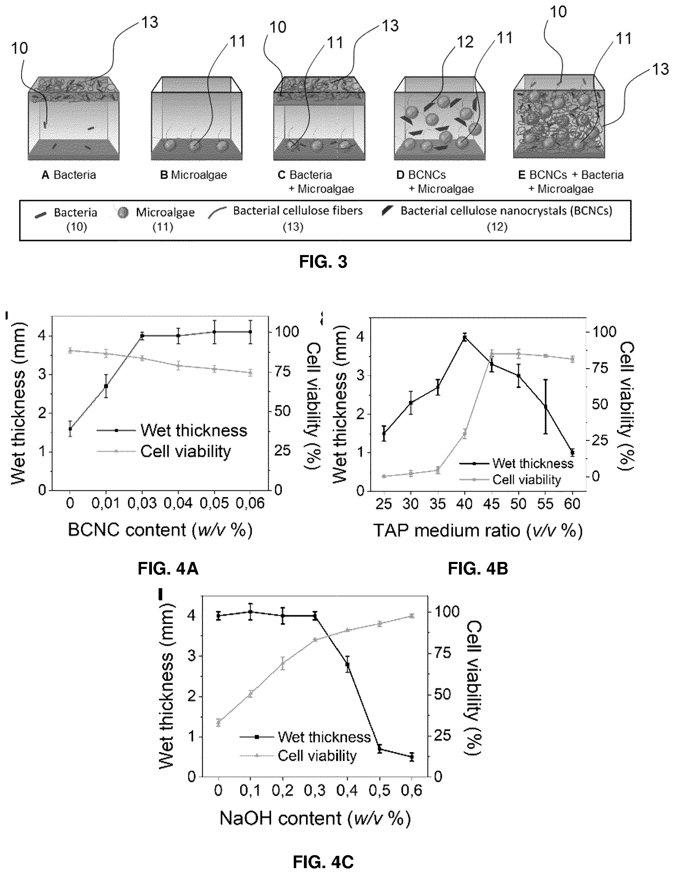

FIG 3A illustrates the status of bacteria 10 in a culture after four days of static incubation: As the production of BC 13 requires oxygen and gas diffusion is limited, the BC 13 is formed as a solid flat pellicle at the air-liquid interface under static incubation. FIG. 3B illustrates the status of microalgae 11 in a culture after four days of static incubation: In the absence of a dispersant, microalgae 11 forms a sediment at the bottom of the jar. The status of a culture of bacteria 10 and microalgae 11 is schematically shown in FIG. 3C after 4 days of static incubation. Due to the algal sedimentation, it is not possible to build enough volume of bacterial cellulose 13 to encapsulate microalgae 11 cells. In consequence, microalgae 11 form a sediment at the bottom and bacteria 10 form a separate pellicle at the air-liquid interface. FIG. 3D illustrates the status of microalgae 11 and BCNC 12 mixture after 4 days of static incubation. When BCNCs 12 as a dispersant are added into the microalgal culture, sedimentation is halted for more than 30 days and microalgae 11 cells remain suspended. FIG. 3E illustrates the status of bacteria 10 and microalgae 11 coculture in the presence of BCNCs (not shown) after 4 days of static incubation. In the presence of a dispersant, the microalgae 11 remain suspended long enough for the bacteria 10 to produce sufficient bacterial cellulose fibres 13 to encapsulate microalgae 10 cells.

The influence of addition of different amounts of bacterial cellulose derived nanocrystals, of different culture medium ratios and NaOH contents, respectively, on sample wet thicknesses and cell viability are shown by the results of Fig. 4A to 4C.

Wet thickness of samples is used here as a benchmark for the production of cellulose. For this purpose, six identical samples of co-cultures comprising *Chlamydomonas reinhardtii* in TAP medium and *Komagataeibacter hansenii* in HS medium with a medium volume ratio of TAP:HS=40:60 and 0.3 w/v% NaOH content are prepared and cultured for 4 days under statically motionless condition, differing only in their bacterial cellulose nanocrystal (BCNC) content, which is used as dispersant. In FIG. 4A, the obtained experimental results are shown. In the absence of BCNC (not according to the invention), a wet thickness of 1.6 ± 0.2 mm is obtained. When the bacterial cellulose nanocrystal content is increased to 0.03 *w*/*v%,* the wet thickness of the engineered living material according to the invention increases to 4.0 ± 0.1 mm. The wet thickness of the engineered living material remains unchanged when the BCNC content is increased above 0.03 *w*/*v*%. Addition of increasing amounts of BCNC continuously reduces the microalgae cell viability. When the BCNCs content is above 0.03 *w*/*v%,* cell viability of the microalgae decreases from 83.7 ± 1.6 % at 0.03 *w*/*v%* to 74.6 ± 2.2 % at 0.06 *w*/*v*%. It is believed that this decrease in cell viability is caused by a decrease in pH value of the culture, which is unfavourable for the viability microalgae cells.

Further, the influen=ce of the composition of mixed culture media on the wet thickness and microalgae cell viability of the engineered living material is demonstrated in FIG. 4B. For this purpose, six identical samples of co-cultures comprising *Chlamydomonas reinhardtii* in TAP medium and *Komagataeibacter hansenii* in HS medium with 0.03 w/v% of bacterial cellulose nanocrystals as a dispersant are prepared and cultured for 4 days under statically motionless condition, only differing in volume ratio of TAP to HS medium. Only the proportion of the TAP medium is specified in percent of a volume. Thereby TAP 25 corresponds to a volume ratio of 25 % TAP medium. Volume shares missing at 100 % correspond to the volume share of HS medium. For this experiment, no alkaline agent is added. In FIG. 4B, the obtained experimental results are shown. When the TAP medium ratio is between 25% (TAP25) to 35% (TAP35), bacteria tended to form bacterial cellulose layers at the air-liquid interface and only a thin layer of microalgae containing bacterial cellulose could form within the liquid, having a wet thickness from 1.5 ± 0.2 mm (TAP25) to 2.7 ± 0.1 mm (TAP35). The microalgae cell viability remains from 0.3 ± 0.3 % (TAP25) to 4.6 ± 2.3 % (TAP35), which is too low for a stable coculture. When the TAP medium ratio is 40% (TAP40), bacterial cellulose forms within the liquid rather than at the air-liquid interface. At this transition point (TAP40), the wet thickness of the engineered living material reached a maximum of 4.0 ± 0.1 mm and the microalgae cell viability increases to 29.9 ± 3.5 % in comparison to TAP35. Further increase of the content of TAP medium reduces bacterial cellulose production due to the limited amount of HS medium available for *Komagataeibacter hansenii.* Further increase of the content of TAP medium therefore decreases wet thickness of the engineered living material from 3.3 ± 0.2 mm (TAP45) to 1.0 ± 0.1 mm (TAP60). Although the cell viability of microalgae cells for TAP45 is higher (85.2 ± 2.9 %) than that of TAP40 (29.9 ± 3.5 %), higher robustness of the engineered living material is obtained with TAP40 as the medium ratio in this experiment.

Also, the influence of the addition of an alkaline agent on the wet thickness and microalgae cell viability of the engineered living material is demonstrated by FIG. 4C. For this purpose, six identical samples of co-cultures comprising 40 % of a liquid culture of *Chlamydomonas reinhardtii* in TAP medium and 60 % of a liquid culture of *Komagataeibacter hansenii* in HS medium with 0.03 w/v% of bacterial cellulose nanocrystals as a dispersant, are prepared and cultured for 4 days under statically motionless condition, only differing in the content of sodium hydroxide (NaOH). FIG. 4C illustrates variations in wet thickness and cell viability of an engineered living material depending on the content of NaOH. The microalgae cell viability increased from 33.3 ± 2.2 % (0 *w*/*v%* NaOH content) to 83.1 ± 0.7 % (0.3 *w*/*v%* NaOH content). The addition of NaOH does not influence the wet thickness (4.0 ± 0.1 mm) of BC-based composites when the NaOH content is not higher than 0.3 *w*/%*.* However, when the NaOH content reaches 0.4 *w*/v%*,* the wet thickness of BC-based composites decreased significantly to 2.8 ± 0.2 mm. This decrease in wet thickness is mainly due to the pH increase from 4.1 (0.3 *w*/*v*%) to 4.3 (0.4 *w*/*v*%) to 5.1 (0.5 *w*/*v*%) and 7.1 (0.6 *w*/*v*%)*,* respectively, which is unfavourable for bacterial cellulose production. In combination with the microalgae cell viability and wet thickness of engineered living material, a NaOH content of 0.3 *w*/*v%* shows best results.

In summary, by considering the trade-off between microalgae cell viability and wet thickness of the engineered living material comprising *Chlamydomonas reinhardtii* and *Komagataeibacter hansenii,* best parameters for cocultivation include a content of 0.03 *w*/*v%* to 0.06 w/v %, specifically of 0.03 w/v %, of BCNCs, a medium volume ratio of 40 % to 50 % TAP medium and 60 % to 50 % HS medium, specifically 40 % TAP and 60 % HS, and a NaOH content of 0.0 to 0.3 *w*/*v* %, specifically of 0.3 w/v %, of NaOH. The beforementioned specific culture conditions provide engineered living materials comprising *Chlamydomonas reinhardtii* and *Komagataeibacter hansenii* with both high cell viability (~ 83 %) and high wet thickness (~ 4.0 mm). The thickness of the engineered living material can exceed 4.0 mm and depends on the dimensions of the culture vessel used. Consequently, the beforementioned engineered conditions are realized within the following embodiment of the method according to the invention.

In one embodiment of the method according to the invention, the provided liquid culture of a first microorganism comprises microalga *Chlamydomonas reinhardtii. A* liquid culture of *Chlamydomonas reinhardtii* microalgae was obtained by culturing in Tris-acetate-phosphate (TAP) medium. To prepare the TAP medium, 25 mL Beijernick salts (15.0 g L⁻¹ NH₄Cl, 4.0 g L⁻¹ MgSO₄·7H₂O, 2.0 g L⁻¹ CaCl₂·2H₂O), 20 mL Tris base (121.1 g L⁻¹), 375 µL phosphate solution (K₂HPO₄: 288 g L⁻¹; KH₂PO₄: 144 g L⁻¹), 1 mL CoCl₂ (1 g L⁻¹), 1 mL glacial acetic acid, and 1 mL trace elements (1) 25mM EDTA-Na₂·2H₂O, (2) 28.5µM (NH₄)₆Mo₇O₂₄, (3) 2mM CuCl₂·2H₂O and 2mM EDTA-Na₂·2H₂O, (4) 2.5mM ZnSO₄·7H₂O and 2.75mM EDTA-Na₂·2H₂O, (5) 6mM MnCl₂ and 6mM EDTA-Na₂·2H₂O, (6) 20mM FeCl₃·6H₂O, 22mM EDTA-Na₂·2H₂O and 22mM Na₂CO₃ were filled up with purified water to 1L and mixed. The prepared TAP medium was then autoclaved at a temperature of 126 °C for use. To prepare microalgae liquid culture, 1 *v*/*v % Chlamydomonas reinhardtii* with an optical desity of 0.5 at 750 nm were inoculated into TAP medium and cultured for 4 days in an incubator (e.g. Panasonic MLR-352-PE) at 25 °C with 3Ls light (800 W) under 18h/6h light/dark conditions.

In the same embodiment of the method according to the present invention, the provided liquid culture of a second microorganism comprises *Komagataeibacter hansenii.* A pellicle comprising bacterial cellulose and *Komagataeibacter hansenii* was obtained by inoculating 1 *v*/*v %* of *Komagataeibacter hansenii* into Hestrin-Schramm (HS) medium containing 5.0 g L⁻¹ tryptone, 5.0 g L⁻¹ yeast extract, 2.7 g L⁻¹ disodium hydrogen phosphate, 1.5 g L⁻¹ citric acid and 20 g L⁻¹ glucose under statically motionless incubation at 30 °C for 4 days. A liquid culture of *Komagataeibacter hansenii* was obtained by separation of liquid supernatant of the 4-day cultured culture prepared under the beforementioned conditions.

Within the same embodiment of the method according to the invention, the provided dispersant comprises bacterial cellulose derived nanocrystals as a dispersant. Bacterial cellulose nanocrystals are obtainable by purification and freeze drying of a wet bacterial cellulose pellicle. A solid bacterial cellulose pellicle harvested at the air-liquid interface of a culture obtained by the procedure described above was purified by boiling within 1 w/v% sodium hydroxide (NaOH) solution for 3 times, followed by refreshing water to remove the unreacted medium within BC until the solid pellicle turns white. Freeze-drying of the purified, wet bacterial cellulose pellicle was carried out using a freeze-drier at -76.6 °C with 23 mT pressure. The freeze-dried pellicle was chopped into flakes with a grinder. The ground bacterial cellulose flakes were added into 60 *wt*% sulfuric acid (aq.) under stirring with an acid/cellulose ratio of 70 mL g⁻¹. Bacterial cellulose nanoparticles form after the hydrolysis procedure at 60 °C, 500 rpm for 1 h. After acidic hydrolysis, the bacterial cellulose nanocrystals were poured into 3 times their volume of purified water to quench the reaction. The bacterial cellulose nanoparticle suspension was then centrifuged for 3 times to remove a majority of the acid. After centrifugation, the bacterial cellulose nanocrystals were resuspended in 100 mL of purified water. To further remove acid residuals, the suspension was transferred into a dialysis tube and the water is changed on a daily basis for the duration of a week. To further increase the stability of bacterial cellulose nanoparticle dispersion, tip sonication was carried out in an ice bath with an effective sonication time of 5 mins, and a total sonication time of 7 min 30 s. The sonicated dispersion was then filtered with an 8 µm pore size filter paper to remove aggregates. The bacterial cellulose nanoparticle dispersion was then autoclaved and stored at 4 °C for use.

In the same embodiment of the method according to the invention, a mixture of the liquid cultures of *Chlamydomonas reinhardtii, Komagataeibacter hansenii* and bacterial cellulose derived nanocrystals, which were prepared as described above, was prepared. For this purpose, a 4-day-cultured microalgae liquid culture was centrifuged at 1780 g for 5 mins at 20 °C and the microalgae solid pellet was resuspended in fresh TAP medium. The supernatant of the 4-day-cultured bacteria culture, the beforementioned microalgae liquid culture and bacterial cellulose nanocrystals as prepared are mixed with an overall ratio of 0.03 *w*/*v %* of bacterial cellulose nanocrystals, 40% based on the volume of TAP medium of the liquid culture of *Chlamydomonas reinhardtii* and 60 % based on the volume of HS medium of the liquid culture of *Komagataeibacter hansenii* were mixed. In this embodiment, 0.3 *w*/*v*% of NaOH was added to the mixture to adjust the pH value.

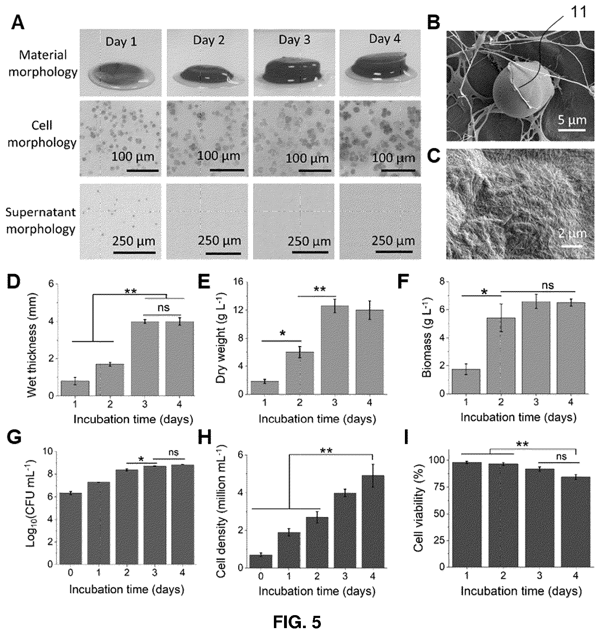

Within the same embodiment, the prepared mixture was cocultured to generate an engineered living material. For doing so, the mixture prepared before was cultured in the coculture solution. Incubation was done in a static incubator (e.g. Panasonic MLR-352-PE) at 25 °C with 3Ls light (800 W) under 18h/6h light/dark conditions. Wet thickness and supernatant morphologies were observed for the first 4 days of incubation in order to quantify growth rate of the engineered living material according to the invention. Observations and results are summarized in FIG. 5A to 5I. Within 4 days of incubation, the symbiotic coculture grows into an engineered living material. FIG. 5A shows the development during days 1 to 4 of cocultivation regarding the material morphology of an engineered living material (photographs), cell morphology of the engineered living material (microscope) and morphology of the supernatant (microscope). Photographs show increasing wet thickness from day 1 to day 4. Microscopic pictures of cell morphology indicate increasing cell density from day 1 to day 4. After 2 days of incubation, all the microalgae cells from the supernatant are trapped inside a network of bacterial cellulose and covered by fibres and *Komagataeibacter hansenii* bacterial cells. FIG. 5B shows a scanning electron microscope image (SEM) of a microalga 11 cell entangled in a fibrous bacterial cellulose network. FIG. 5C shows a scanning electron microscope image (SEM) of a microalgal cell covered by bacteria and bacterial cellulose fibres. In order to quantify the material growth rate, wet thickness of the engineered living material was measured day by day. FIG. 5D shows the development of the wet thickness of the engineered living material during the first four days of cocultivation. The wet thickness increased from 0.8 ± 0.2 mm (day 1) to 1.7 ± 0.1 mm (day 2) and 4.0 ± 0.1 mm (day 3) and 4.0 ± 0.2 mm (day 4), respectively. The wet thickness value reached a maximum after 4 days of incubation. FIG. 5E shows the development of dry weight of the engineered living material during the first four days of cocultivation. The dry weight values increased from 1.8 ± 0.3 g L⁻¹ (day 1), to 6.0 ± 0.8 g L⁻¹ (day 2) and 12.6 ± 1.0 g L⁻¹ (day 3) and 12.0 ± 1.3 g L⁻¹ (day 4), respectively. FIG. 4F shows the development of biomass of the engineered living material during the first four days of cocultivation. The biomass values increased from 1.8 ± 0.4 g L⁻¹ (day 1) to 5.4 ± 1.0 g L⁻¹ (day 2), 6.6 ± 0.5 g L⁻¹ (day 3), 6.5 ± 0.3 g L⁻¹ (day 4), respectively. In combination with the overall dry weight values and biomass values, it can be concluded that bacterial cellulose production mainly happened on day 3 and remained unchanged on day 4. In the final grown bacterial cellulose-based bio-composites (dry state), the overall bacterial cellulose content is 45.8 % while the biomass content is over 54.2%. By calculating the bacterial cellulose production yield, it could be concluded, that over 94.5 % of the bacterial cellulose was produced within 4 days of incubation, with an overall bacterial cellulose production yield of 5.2 g L⁻¹, which is higher than pure BC (4.3 g L⁻¹). To understand the cell behaviour during the coculture process, the cell viability of *Komagataeibacter hansenii* bacteria was determined. FIG. 5G shows the logarithmic development of colony forming units (CFU) of bacteria within an engineered living material during the first four days of cocultivation, which increased from 6.3 ± 0.1 Log(CFU mL⁻¹) (day 0) to 7.3 ± 0.01 Log(CFU mL⁻¹) (day 1), 8.4 ± 0.1 Log(CFU mL⁻¹) (day 2), reaching 8.7 ± 0.03 Log(CFU mL⁻¹) (day 3) and 8.8 + 0.01 Log(CFU mL⁻¹) (day 4). FIG. 4H shows the development of the cell density of *C. reinhardtii* microalgae in the engineered living material during the first four days of cocultivation. Cell density increased from 0.7 ± 0.1 million mL⁻¹ (day 0) to 1.9 ± 0.2 million mL⁻¹ (day 1), 2.7 ± 0.3 million mL⁻¹ (day 2), 4.0 ± 0.2 million mL⁻¹ (day 3), 4.9 ± 0.6 million mL⁻¹ (day 4). FIG. 5I shows the development of cell viability of *C. reinhardtii* microalgae in the engineered living material during the first four days of cocultivation. Cell viability decreased slightly from 98.0 ± 1.1 % (day 1), 96.6 ± 1.4 % (day 2), 91.8 ± 1.9 % (day 3), 84.3 ± 2.3 % (day 4). Within 4 days of incubation, cell viability of *C. reinhardtii* microalgae remained higher than 80 %.

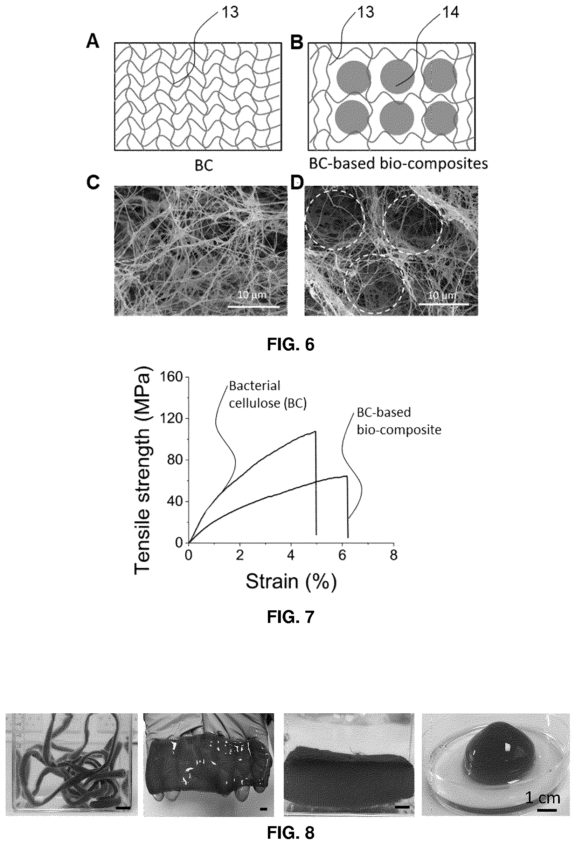

Due to the presence of cells of a first microorganism, within the engineered living material, the bacterial cellulose fibre density within a comparable cross-section area is smaller than the fibre density of pure bacterial cellulose. FIG. 6A illustrates schematically the fibre density of bacterial cellulose 13 in a conventional culture comprising solely a second microorganism (not shown). FIG. 6B shows the fibre density of bacterial cellulose 13 within an engineered living material according to the invention, comprising a first microorganism 14 (shown as circles) and a second microorganism (not shown). FIG. 6C shows a scanning electron microscope (SEM) image of the fibre density of a dried sample of bacterial cellulose grown in a conventional culture comprising solely a second microorganism. Conventionally grown bacterial cellulose has a higher fibre density, as no space is required for cells of a first microorganism. FIG. 6D shows a scanning electron microscope (SEM) image of the fibre density of a dried sample of an engineered living material according to the invention. The white circles indicate free spaces, which were occupied by cells of a first microorganism before drying. This results in a reduced fibre density and increased porosity of the respective dried material.

To compare mechanical properties of the engineered living material with conventionally grown bacterial cellulose, tensile tests were performed. As the water content influences the accuracy of tensile results, to fairly compare the mechanical properties, tensile testing was performed under dry conditions. FIG. 7 shows tensile properties of a dried sample of bacterial cellulose grown in a conventional culture comprising solely a second microorganism and of a dried sample of an engineered living material according to the invention (BC-based bio-composite). After drying, the engineered living material has a higher elongation at break value (6.7 ± 0.2 %) and a lower tensile strength value (65.5 ± 2.2 MPa) compared to that of pure bacterial cellulose (elongation at break: 4.8 ± 0.2 %; tensile strength: 118.9 ± 6.4 MPa). Even though at a dry state, the material shows competitive mechanical robustness.

The engineered living material according to the invention has a high mechanical robustness, allows for an increase in overall bacterial cellulose production yield of 5.2 g L⁻¹, which is higher than the production yield of pure bacterial cellulose (4.3 g L⁻¹) and can grow to flexible shapes with thicknesses of several centimetres. FIG. 8 illustrates four examples of geometries of the engineered living material demonstrating accessible shape diversity.

### Material characterization

The morphology of BC-based bio-composites was monitored with a Keyence VHX-7000 digital optical microscope under transmission mode. The wet thickness of BC-based bio-composites was measured with a calliper.

To measure the microalgae cell density and cell viability within BC-based bio-composites, BC-based bio-composites were transferred from 12-well plate to 50 mL falcon tube, followed by the addition of 50 µL cellulase from *Trichoderma reesei* (aqueous solution, ≥700 units g⁻¹). The falcon tubes were then transferred into an INFORS HT shaking incubator at 30 °C, 250 rpm for 4 hours. After the cellulase treatment, BC-based bio-composites were dissolved and the cells were suspended in the liquid. The cell suspension was subsequently centrifuged with a Hettich ROTINA 38R centrifuge machine at 1780 g for 5 mins at 20 °C to remove the cellulase and liquid medium. The solid cell pellet was then resuspended into 4 mL TAP medium for use. To measure the microalgae cell density, 10 µL of resuspended liquid was added into a Marienfeld Neubauer hemocytometer (0.100 mm in depth, 0.0025 mm² area) and the microalgae cell density was checked with a Keyence VHX-7000 digital optical microscope under *400 magnification. To measure the microalgae cell viability, the resuspended liquid and 0.4 % Trypan Blue solution (Gibco) with a volume ratio of 1:1 were mixed and this staining process was kept for 5 mins. Afterwards, the mixture was centrifuged with a JENCONS -PLS 24D Spectrafuge centrifuge machine for 5 mins at *13 g, 20 °C. The trypan blue dye was then removed and same amount of TAP medium was added to resuspend microalgae cells. Next, 10 µL of this stained resuspended cell suspension were added into a Marienfeld Neubauer hemocytometer (0.100 mm in depth, 0.0025 mm² area) and the microalgae cell viability was checked with a Keyence VHX-7000 digital optical microscope under 400-fold magnification. The dead microalgae cells showed a blue colour while the living microalgae cells showed a green colour. Microalgae cell viability was calculated according to the number of living cells divided by the overall microalgae cell number. To measure the dry weight of BC-based bio-composites, the 4-day-old BC-based bio-composites were transferred from 12-well plates into falcon tubes, followed by drying in an oven at 40 °C for 4 days. To measure the biomass of BC-based bio-composites, the BC-based bio-composites from 12-well plate were transferred to 50 mL falcon tube, followed by the addition of 50 µL cellulase from *Trichoderma reesei* (aqueous solution, ≥700 units g⁻¹). The falcon tubes were then transferred into an INFORS HT shaking incubator at 30 °C, 250 rpm for 4 hours. After the cellulase treatment, BC-based bio-composites were dissolved and the cells were suspended in the liquid. The cell suspension was then centrifuged with a Hettich ROTINA 38R centrifuge machine at 1780 g for 5 mins at 20 °C to remove the cellulase and liquid medium. The cell pellets were washed by resuspending them into water and centrifuging again for 3 times. The final washed cell pellets (bacteria and microalgae) were dried in an oven at 40 °C for 4 days. The BC yield within the BC-based bio-composites can be calculated with the following equation: $Yield of BC = \frac{{m}_{dryweight} - {m}_{biomass} - {m}_{BCNCs mass}}{{m}_{dryweight} - {m}_{biomass}} \times 100 %$ where m _{dryweight} is the dry weight of BC-based bio-composites, including the BCNCs, microalgae, bacteria and freshly formed BC; m _{biomass} is the weight for bacteria and microalgae; m _{BCNCs mass} is the initial addition amount of BCNCs within the coculture solution.

To measure the bacteria viability within BC-based bio-composites, the cocultured BC-based bio-composites with different incubation time were taken from 12-well plate into 50 mL falcon tube and the above-mentioned cellulase treatment was repeated. Afterwards, the *Komagataeibacter hansenii* bacterial viability was assessed with colony-forming unit (CFU) measurements. Briefly, the bacterial pellet was resuspended in the same initial volume of saline (0.9% w/v NaCl). Dilutions of 10⁰-10⁻⁸ were prepared, followed by spotting 10 µL of each dilution on HS-agar plates (supplemented with 2% *v*/*v* acetic acid). The HS-agar plates were then cultured at 30 °C for 3 days, and the number of colonies was enumerated and the log₁₀ (CFU mL⁻¹) was calculated.

### Revivability and longevity of BC-based bio-composites

To check if the BC-based bio-composites can be revived, the 4-day-old BC-based bio-composites were transferred into a 50 mL falcon tube with 30 mL TAP medium. The falcon tubes were cultured in a Panasonic MLR-352-PE incubator at 25 °C with 3Ls light (800 W) under 18h/6h light/dark conditions for 1, 4 and 7 days. To check the longevity of BC-based bio-composites, the 4-day-old BC-based bio-composites were transferred into an empty 50 mL falcon tube, then the lid was closed and the falcon tube was kept at ambient lab conditions for 1 month. Afterwards, 30 mL TAP medium were added into the falcon tube for revival.

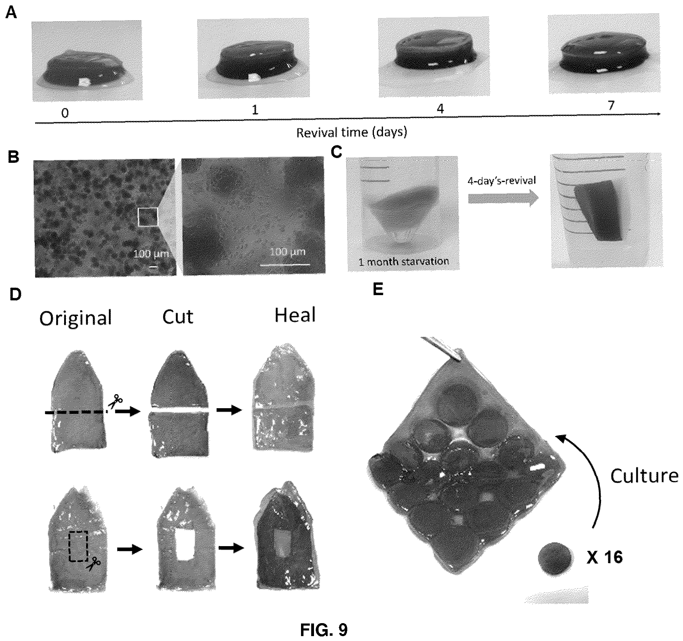

The BC-based bio-composites could be revived by being immersed into TAP medium. After 1 day of revival, the BC-based bio-composites turned greener, while longer revival (4 and 7 days) time had little influence on the greenness. Revived BC-based bio-composites are shown in Fig. 9A. The biomass values of the revived samples increased from 6.5 ± 0.3 g L⁻¹ (day 0 revival) to 10.2 ± 0.4 g L⁻¹ (day 1 revival), 10.8 ± 0.9 g L⁻¹ (day 4 revival), 10.8 ± 0.4 g L⁻¹ (day 7 revival). Therefore, the revival happened mainly on the first day of immersion into the TAP medium. Fig. 9B shows optical microscope images of BC-based bio-composites (wet) after 4 days of revival, during which microalgae cells form aggregations.

To assess the longevity of the BC-based bio-composites, we put a 4-day cocultured BC-based bio-composite material into an empty falcon tube. No additional medium was added only the water retained by the BC-bio composite and the falcon tube was sealed and kept at ambient laboratory conditions for 1 month. The 30-day old sample is shown in Fig. 9C. To check the status of the algae in bio-composite TAP medium was added into the falcon. The 30-day old sample became green after 4 days of revival, which is also shown in Fig. 9C, representing that this material can survive at least 1 month without adding any nutrients. This long-time survivability is mainly due to the high water-holding capacity of BC, which can provide sufficient nutrient for microalgae cells to survive within the material. This provides the advantage to ship the material over long distances, or a use in space colonization, making it potentially being used to produce sustainable energy in places where plants don't grow well.

### Self-healing properties of BC-based bio-composites

Self-healing properties of an engineered living material according to the invention are illustrated in Fig. 9D. To check the self-healing properties of BC-based bio-composites, the BC-based bio-composites were prepared by adding 100 mL coculture solution into a 50 ml cell culture flask, the flasks were cultured in a Panasonic MLR-352-PE incubator at 25 °C with 3Ls light (800 W) under 18h/6h light/dark conditions for 4 days. Afterwards, the BC-based bio-composites were taken out of the incubation flask and damages were created by cutting the samples into 2 pieces or digging the sample with a rectangular hole, which is shown in Fig. 9D. The damaged samples were transferred back to a fresh sterilized cell culture flask, and healed by adding 50 mL coculture solution into a 50 ml cell culture flask. Then flasks were incubated again under statically motionless condition in a Panasonic MLR-352-PE incubator at 25 °C with 3Ls light (800 W) under 12h/12h light/dark conditions for 4 days. Fig. 9D shows the damaged BC-based bio-composites can be healed after incubation.

### Scalability of BC-based bio-composites

Scalability of the engineered living material according to the present invention is demonstrated in Fig. 9E. To prepare scalable BC-based bio-composites, two approaches were applied: In the first approach, 300 mL coculture solution were poured into a 30*30 cm glass vessel and the vessel was cultured under statically motionless condition in a Panasonic MLR-352-PE incubator at 25 °C with 3Ls light (800 W) under 18h/6h light/dark conditions for 4 days. In the second approach, several building blocks were prepared by growing 4 mL coculture solution in 12-well plates in static in an Panasonic MLR-352-PE incubator at 25 °C with 3Ls light (800 W) under 18h/6h light/dark conditions for 4 days, followed by further growing these building blocks into a larger piece into a 50 ml cell culture flask with the addition of 50 mL coculture solution under same conditions. The building blocks could be healed successfully by adding fresh coculture liquid mixture to form fresh tissues between the junction points. Such property has the advantage to be exploited for transformation or joining of differently produced blocks into more complicated 3D-shapes by further incubation of pre-grown building blocks. Alternatively, to get a scalable and homogeneous part, the material can be grown directly in a larger vessel. Successful joining of 16 single blocks, which grew into a large, single piece, is shown in Fig. 9E.

### Tensile properties of BC-based bio-composites

To check the tensile properties of BC and BC-based bio-composites, 100 mL bacterial monoculture or coculture solution were added into a 50 ml cell culture flask, then the flasks were incubated under statically motionless condition in a Panasonic MLR-352-PE incubator at 25 °C with 3Ls light (800 W) under 18h/6h light/dark conditions for 4 days. Afterwards, the samples were taken out of the flasks, put overtop of flasks and dried in Class II cabinet for 5 days. After drying, samples were cut into a dimension of 60 mm* 10 mm*0.05 mm (Pure BC), 60 mm* 10 mm*0.1 mm (BC-based bio-composites) for tensile testing. The tensile tests were performed using an Instron universal testing machine with a 10 kN load cell and 1 kN grips. The measuring distance between the clamps was 20 mm, and the samples were tested with a loading rate of 5 mm min⁻¹. At least three specimens per group were measured for the data presented here.

### List of reference signs

- 10: Bacteria
- 11: Microalgae
- 12: Bacterial cellulose nanocrystals (BCNC)
- 13: Bacterial cellulose (BC)
- 14: First microorganism

### Cited non-patent literature

[1] V.-D. Girard, J. Chaussé, P. Vermette. Bacterial cellulose: A comprehensive review. J. Appl. Polym. Sci. 15, 141 (2024).
[2] J.-T. Hsieh, M.-J. Wang, J.-T. Lai, H.-S. Liu. A novel static cultivation of bacterial cellulose production by intermittent feeding strategy. J. Taiwan. Inst. Chem. Eng. 63, 46, (2016).
[3] Caro-Astorga, J., Walker, K. T., Herrera, N., Lee, K. Y. & Ellis, T. Bacterial cellulose spheroids as building blocks for 3D and patterned living materials and for regeneration. Nat Commun 12, 5027 (2021).
[4] Lee, D., Gutowski, I. A., Bailey, A. E., Rubatat, L., de Bruyn, J. R., Frisken, B. B. Investigating the microstructure of a yield-stress fluid by light scattering. Phys. Rev. E 83, 031401 (2011).

## Claims

1. A method for cocultivation of microorganisms, in particular for producing bacterial cellulose and biomass, including the steps of:
(a) Providing a liquid culture of at least a first microorganism which is an oxygen producing microorganism;
(b) Providing a liquid culture of at least a second microorganism which is capable of producing bacterial cellulose;
(c) Providing a dispersant;
(d) Preparing a mixture of the liquid cultures of step (a), step (b) and of the dispersant provided in step (c);
(e) coculturing the mixture obtained in step (d) to generate an engineered living material.

2. The method according to claim 1, wherein during step (e) of coculturing, the mixture is kept statically motionless.

3. The method according to claim 1 or 2, wherein the first microorganism is a photosynthetic microorganism.

4. The method according to claim 3, wherein the first microorganism is at least one of a microalgae, preferably eukaryotic microalgae, such as *Phaeodactylum tricornutem,* or *Chlamydomonas reinhardtii,* or one of the Chlorella genus of the division Chlorophyta, such as *Chlorella vulgaris,* or *Chlorella sorokiniana,* or a bacteria, such as cyanobacteria, preferably from Phylum Cyanobacteria, such as Chroococales, Nostocales, or Spirulinales, or a combination thereof.

5. The method according to at least one of the preceding claims, wherein the second microorganism is at least one from the group consisting of *Acetobacter xylinum, Komagataeibacter hansenii,* and *Acetobacter pasteurianus and combinations thereof.*

6. The method according to at least one of the preceding claims, wherein the dispersant is in the form of colloidal particles having a particle size of 1.0 µm or less, and provides after step (d) in particular for a fluid having a low yield stress.

7. The method according to claim 6, wherein the dispersant is in the form of charged particles comprising ionic groups on their surface, and/or the dispersant comprises inorganic particles, for example silica particles, and/or the dispersant comprises organic particles, preferably particles of a polymeric compound, and/or the dispersant is a polysaccharide, preferably cellulose nanocrystals and even more preferably bacterial-cellulose derived cellulose nanocrystals or plant derived cellulose nanocrystals.

8. The method according to at least one of the preceding claims, wherein the second organism in step (b) is provided in a further liquid culture medium, the further liquid culture medium being the different from the liquid culture medium of step (a).

9. The method according to at least one of the preceding claims, wherein in step (d), the concentration of the dispersant is adjusted to the viability of the first and/or second microorganism.

10. The method according to at least one of the preceding claims, wherein in step (d), a ratio of liquid culture media of the liquid culture of step (a) and of the liquid culture of step (b) is adjusted to the viability of the first and/or second microorganism.

11. The method according to at least one of the preceding claims, wherein in at least one of steps (d) and (e) a pH adjusting additive is added, in particular one selected from the group consisting of buffers, acids, bases and mixtures thereof.

12. The method according to at least one of the preceding claims, wherein in at least one of steps (d) and (e) an alkaline agent is added, particularly NaOH, to adjust a pH value of the mixture to the range of 4.0 to 7.5, preferably 4.5 to 6.5, and even more preferably 5.0 to 6.0.

13. An engineered living material comprising
a first microorganism which is an oxygen producing microorganism,
a second microorganism which is capable of producing bacterial cellulose, and
a dispersant.

14. The engineered living material according to claim 21, obtainable by a method according to at least one of claims 1 to 20, and/or wherein the engineered living material has a bulk thickness of at least 1 mm, preferably of at least 2 mm.

15. A bioreactor comprising the engineered living material according to claim 13 or 14 for the production of compounds of interest, preferably selected from the group consisting of bacterial cellulose, nanocellulose, antioxidants, vitamins, particularly vitamin B12, pigments, particularly β-carotene and copper-chlorophyllin pigments, Eicosapentaenoic acid (EPA), docosahexaenoic acid (DHA), proteins, biochar and/or agricultural bio-stimulants and combinations thereof.

16. Production method for producing a compound of interest within the engineered living material according to claim 13 or 14, in particular in a bioreactor according to claim 15, wherein the compound of interest is obtained from the engineered living material, in particular by subjecting the engineered living material to a step of macerating, extracting, separating and/or purifying components of the engineered living material.

17. Bacterial cellulose, obtainable from the engineered living material according to claim 13 or 14.
